# EUROPEAN PATENT APPLICATION

(11) **EP 3 838 231 A1**
(43) Date of publication of application: **23.06.2021**
(21) Application number: 19216470.5
(22) Date of filing: 16.12.2019
(51) Int. Cl.: A61F 2/40, A61F 2/30, A61F 2/38, A61F 2/46, B29K 23/00, B29K 623/00

(54) **COMPONENT FOR A JOINT REPLACEMENT**

(71) Applicant: Waldemar Link GmbH & Co. KG, 22339 Hamburg (DE)
(72) Inventor: DALLA PRIA, Paolo, 33100 Udine (IT); SCHULTZ, Thomas, 21365 Adendorf (DE)
(74) Representative: Hoffmann Eitle

(57) **Abstract**

The disclosure relates to a replacement member (1, 100) for a shoulder joint replacement comprising an attachment face (3) on one side of the member (1, 100), a concave joint surface (5) on a side of the replacement member (1, 100) opposite to the side of the attachment face (3), and a circumferential face (7) connecting the attachment face (3) and the joint surface (5). A portion of the concave joint surface (5) is connected to at least a portion of the circumferential face (7) via a chamfered or rounded edge (9).

## Description

### FIELD OF THE INVENTION

The present invention generally relates to joint replacements and in particular to shoulder joint replacements. More particularly, the present invention relates to a replacement member with a concave joint surface.

### BACKGROUND

A native joint may undergo degenerative changes for a variety of reasons, for instance arthritis. Also, a joint may be fractured or otherwise be damaged by an external force affecting the functionality of its joint surfaces, in particular the hyaline cartilage forming these surfaces. If such a joint is significantly degenerated or damaged, it may become necessary to replace the native joint with a joint replacement.

Such a replacement may begin with a surface replacement and/or a hemi-arthroplasty, both being a partial replacement of a native synovial joint. This is generally only a temporary solution that will likely be followed by total joint replacement. Further, the joint replacement is also subject to wear and may have to be replaced, a so-called revision.

For example, a total shoulder replacement comprises a humeral component and a glenoid component. However, for a hemi-arthroplasty, the humeral component articulates against the native glenoid cavity.

In an anatomic arthroplasty, the structure of a joint replacement resembles an anatomical shoulder with a convex anatomic ball head fixed to the humerus and a concave anatomic glenoid component fixed to the scapula.

Typically, the glenoid component comprises an anchoring portion for insertion into the scapula and a joint portion that provides a joint surface which may be a convex head in case of reverse arthroplasty or a convex cup in case of an anatomic arthroplasty.

Similar to the knee joint, the shoulder joint is primarily stabilized by the ligaments and muscles surrounding this joint. Thus, in any of the surgical techniques mentioned above it is important to keep as much soft tissue intact as possible for the stability of the treated joint. In particular the shoulder joint is prone to luxation and even more so after surgery.

Further, the following problems have been observed with joint replacements and in particular with shoulder joint replacements. Generally, in anatomic shoulder replacements, extreme joint deflection can be the cause of an impingement between the humerus or the humeral component and the rim of the glenoid component (prosthetic glenoid). Such an extreme joint deflection can also include an impingement of tissue between the glenoid and the humerus or the humeral component.

In order to overcome these limitations, reverse shoulder prostheses with eccentric glenospheres have been developed in order to extend the range of motion for adduction to reduce the risk of impingement. However, impingement during adduction may still occur and may cause discomfort to the patient. Also, there is still a risk of dislocation of the shoulder joint, in particular in case of thin patients when holding their arms close to their torso.

Such an impingement is particularly prone to occur at the caudal portion of the shoulder joint replacement during adduction. In order to mitigate this risk, the effective range of motion of the joint is reduced compared to the range of motion of a native joint.

These problems have been addressed by EP 1 064 890 B1. EP 1 064 890 B1 is directed to a reverse shoulder joint and proposes a glenoid, for which the center of rotation of the articulation head is offset downwards in relation to the median plane and to the center of the anatomical glenoid cavity in order to lower the articulation head. Further, the articulation head has on the side of the external pillar of the scapula an extension able to increase the extent of rotation in adduction.

However, there remains a problem that prior art does not provide an anatomical shoulder joint replacement which allows for adduction to an extent comparable to the native range of motion.

### SUMMARY

In view of the above, it is therefore an objective of the present disclosure to provide a shoulder joint replacement that allows for an increased range of motion in comparison to prior art devices, especially an increased range of motion in adduction.

This objective is addressed by a joint replacement member according to independent claim 1, wherein the claims dependent thereon relate to preferred embodiments.

More specifically, the present disclosure provides a replacement member for a shoulder joint replacement, wherein the replacement member comprises an attachment face on one side of the member, a concave joint surface on a side of the member opposite to the side of the attachment face, and a circumferential or peripheral face connecting the attachment face and the joint surface. In the replacement member, a portion of the concave joint surface is connected to at least a portion of the circumferential face via a chamfered or rounded edge.

As used herein, the term "adduction" is used to describe a movement of the components of a joint or joint replacement in the coronal plane in which the angle enclosed by the components becomes smaller. Correspondingly, the term "abduction" is used to describe a movement in the coronal plane in which the angle enclosed by the components becomes larger.

The replacement member comprises a joint surface that is configured to movably engage with a corresponding joint surface of a counterpart member. The joint surface may be a surface recreating the glenoid cavity, whereas the corresponding joint surface of a counterpart member may in this case be a ball joint surface of a native or artificial humerus head.

This ball joint configuration allows for the counterpart member to be rotated in the joint cavity towards the chamfered or rounded edge, while even in an extreme or innermost position maintaining a clearance between the replacement member and the counterpart member. At the same time, the material of the replacement member at the chamfered or rounded edge provides support to the concave joint surface at this position. This support improves longevity of the joint surface due to an enhanced load distribution.

Further, if the rotator cuff is intact, such as in a younger patient, the anatomical configuration of a shoulder replacement is generally the preferred choice of implant. In case of this anatomical configuration, the desired position of the glenoid replacement is in the middle or slightly cranial in relation to the native glenoidal joint surface. An implantation of the glenoid replacement member including a chamfered or rounded edge has the advantage to at least delay impingement if the placement of the glenoid replacement is too far in the cranial direction.

For the anatomical configuration, a caudal position is avoided because in extreme adduction, the humerus may abut against the glenoid replacement. This bears the risk of osteolysis and of an increase in wear. However, such a caudal position of the glenoid replacement is generally used for the reverse configuration of a shoulder joint replacement. In both cases, the rotator cuff becomes weaker over time and the humerus tends to move cranially. Here too, impingement is at least delayed due to the chamfered or rounded edge of the replacement member.

The chamfered or rounded edge extending between the concave joint surface and the circumferential face has a length in a circumferential direction of the concave joint surface and circumferential face and a width substantially perpendicular to this direction. Since a portion of the concave joint surface is connected to the circumferential face via a chamfered or rounded edge, the length of the chamfered or rounded edge is shorter than the circumferential length of circumferential face (and of the concave joint surface).

The width of the chamfered or rounded edge preferably tapers towards both ends in the longitudinal direction of this edge. In this case, the chamfered or rounded edge has a maximum width between these ends and at least one of these ends has a minimum width.

Preferably, the chamfered or rounded edge preferably has a maximum width of at least 3 mm, at least 4 mm, at least 5 mm, at least 6 mm, or at least 7 mm and/or a minimum width of at maximum less than 3 mm, less than 2 mm, less than 1 mm, or less than 0,7 mm.

In case of a rounded edge, the rounded edge may have a convex or concave profile.

Preferably, the chamfered or rounded edge is configured to be located at an adductive portion of the joint replacement when the replacement member is implanted in a body. In other words, the chamfered or rounded edge is arranged at the side of the concave joint surface the humerus moves to or is close to during adduction.

The joint surface as seen in plan view preferably has an oblong shape (even more preferably with a curved circumference for example an elliptical or oval shape), wherein the chamfered or rounded edge is located at (only) one of the ends in the longitudinal direction. This end is to be arranged caudally during implantation, i. e. at the adductive portion.

When the replacement member of the shoulder joint replacement is arranged in a patient such that the chamfered or rounded edge, which connects the concave joint surface and the portion of the circumferential face, faces downwardly, i.e., caudally, a clearance will remain between the caudal part of the replacement member and the corresponding counterpart member. This allows for soft tissue to be accommodated within the clearance even in an innermost adducted position such that the adduction movement of the arm will not be limited by soft tissue being impinged between the replacement member (or the surrounding bone tissue) and the surface of the humerus part of the replacement.

Preferably, the concave surface face has a curved profile, the curved profile being spherical, ellipsoidal, or ovoid shaped.

These configurations, as all concave bulge-like recesses, provide all three rotational degrees of freedom to the joint replacement that are found in a native joint. For this reason, a spherical profile is particularly preferred since it may provide these degrees of freedom using surface contact.

Further, if a large difference between the diameter of the concave surface face and the diameter of the convex joint member is present, this allows for supero-inferior and antero-posterior translational degrees of freedom between the joint members. In particular a dimensional mismatch between the diameters of the head's joint surface (preferably made of metal) and the glenoid's joint surface (preferably made of polymer) enables a limited translation between these two replacement members. For example, the mismatch between diameters may be in a range of 4 to 16 mm and preferably in a range of 6 to 12 mm.

Preferably, the attachment face is configured so as to fixedly attach the member to bone tissue or another replacement member.

With this configuration, a proper fixation of the replacement member in the native surrounding tissue can be achieved.

If the attachment face is configured to fixedly anchor the replacement member to bone tissue, the attachment face is preferably configured for bone ingrowth. For this, the attachment face may comprise a coating (applied using a technical process). Another technique for anchoring the replacement member is via bone cement (in particular on PMMA basis). Here the attachment face is configured to act as an interface to the bone cement. A skilled person will appreciate that the application of bone cement does not represent a technical coating but is instead acting as an adhesive and/or filler in order to attach the replacement member to adjacent bone tissue.

The attachment face may be configured to be fixedly attached to another replacement member, i.e. it is anchored indirectly to the skeleton of the patient. This configuration facilitates the exchange of the replacement member, such as for replacing the joint surface during a revision.

The member may be a glenoid replacement member or a humeral replacement member.

The configuration of the shoulder replacement member may be implemented either for the case of an anatomical replacement as a glenoid or in the case of a reverse replacement as a humeral replacement, i.e. a replacement replacing the joint surface of the humeral side. In both cases, the effect of maintaining a clearance even in an innermost adducted position can be achieved.

Preferably, the section forming the joint surface comprises Vitamin E and ultra-high-molecular-weight polyethylene (UHMWPE).

Such a material has shown to have beneficial mechanical properties for acting as a joint surface. This includes an excellent biocompatibility. Alternatively, it is also possible to use UHMWPE alone or in combination with additional or other additives.

Preferably, the concave joint surface is configured to engage with a mating joint replacement member, which allows for a smooth and natural behavior of the joint replacement.

It is also disclosed a shoulder joint replacement. The shoulder joint replacement comprises a joint replacement member having the features described above and a mating joint replacement member. In an assembled state, the member and the mating joint replacement member engage via a portion of the convex joint surface of the member.

As explained above, this assembly allows to increase the range of motion of an implanted joint replacement since there will remain a clearance between the member and the mating joint replacement member. This clearance prevents impingement from occurring.

There is also disclosed a shoulder joint replacement comprising a first replacement member, in particular a replacement member having the features described above, and a second replacement member. In this shoulder joint replacement, the first replacement member comprises an attachment face on one side of the member, a concave joint surface on a side of the replacement member opposite to the side of the attachment face, and a circumferential face connecting the circumferential attachment face and the joint surface. The second replacement member comprises a convex joint surface, the convex joint surface forming a ball joint with the concave joint surface of the first replacement member, and an outer surface distal and adjacent to the convex joint surface. The concave joint surface is partly connected to the circumferential face via a chamfered or rounded edge.

As a result of the chamfered or rounded edge, a clearance is provided between the first replacement member and the second replacement member when the two replacement members are arranged relatively to each other in adduction. In particular the outer surface of the second replacement member is arranged such that even in the innermost adduction position a clearance between the joint surface and the outer surface is ensured.

This configuration prevents the outer surface of the second replacement member from contacting the joint surface of the first replacement member in an adduction position of the shoulder joint replacement. Thus, impingement is avoided. This avoidance of contact of the chamfered or rounded edge by avoidance of an impingement reduces the wear of the glenoid component with such a design. Additionally, this saves the patient from unnecessary pain caused by such an adducted position and, thus, provides the range of motion the patient is used to in this position.

### BRIEF DESCRIPTION OF THE DRAWINGS

The following figures illustrate preferred embodiments of the present invention. These embodiments are not to be construed as limiting but merely for enhancing the understanding of the invention in context with the following description. In these figures, same reference signs refer to features throughout the drawings that have the same or an equivalent function and/or structure. A repetitive description of these components is generally omitted for reasons of conciseness.
Fig. 1 is a perspective view of a joint replacement member according to an embodiment.
Fig. 2A is a perspective view of a joint replacement member according to an embodiment.
Fig. 2B is a perspective view of an embodiment of an intermediate member for the joint replacement of Fig. 2A.
Fig. 2C shows the joint replacement member of Fig. 2A and the intermediate member of Fig. 2B in an assembled state.
Fig. 2D shows a side-view of the replacement member illustrated in Fig. 2A.
Fig. 2E shows an assembly of a replacement member and an intermediate member according to another embodiment.
Fig. 3A, 3B and 3C show a replacement member according to yet another embodiment which is to be mounted directly to surrounding tissue.

### DETAILED DESCRIPTION

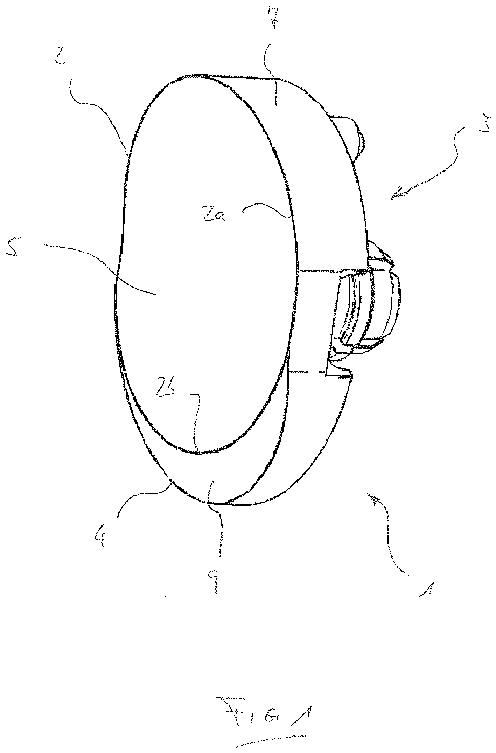

Fig. 1 is a perspective view of an exemplary embodiment of a joint replacement member 1. The joint replacement member 1 comprises an attachment face 3 on one side (hidden in the view in Fig. 1). The attachment face 3 can either be a face that is configured to be directly anchored to the surrounding tissue or can be a face that is to be attached to an intermediate member, which is then anchored to the surrounding tissue.

The joint replacement member 1 shown in Fig. 1 further comprises a concave joint surface 5 on a side of the replacement member 1 opposite to the side of the attachment face 3, said concave joint surface 5 being configured to engage with a mating convex joint replacement member.

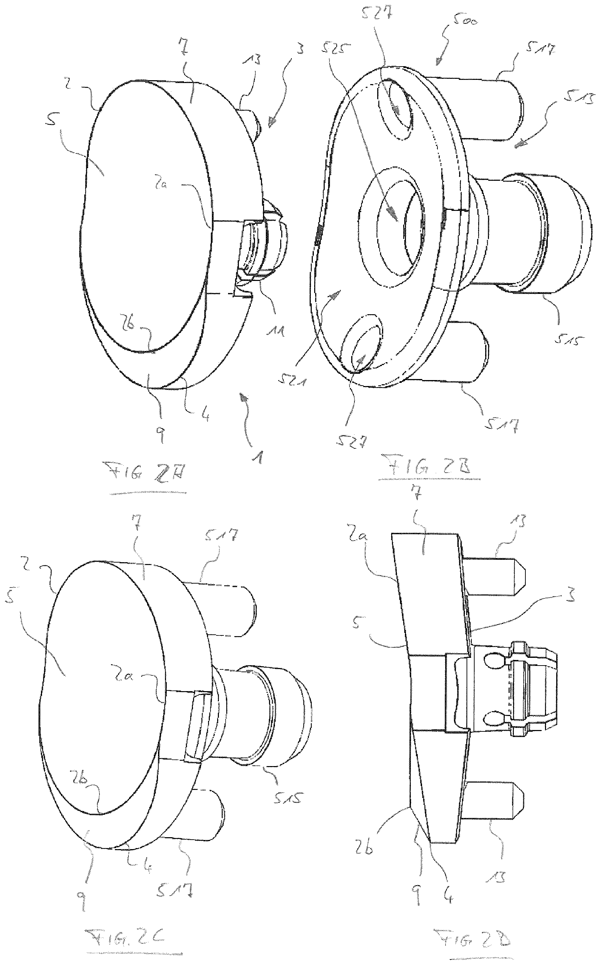

The replacement member 1 further comprises a circumferential face 7 which circumferentially connects the attachment face 3 and the joint surface 5. A portion of the concave joint surface 5 is connected to a portion of the circumferential face 7 via a chamfered or rounded edge 9. In the exemplary embodiment of the figures, the edge 9 is a chamfered edge (see Fig. 2D). As described above, this edge prevents impingement from occurring and an enhanced support.

A plurality of edges is formed in the embodiment of the replacement member 1: a first edge 2, which surrounds the joint surface 5 along its entire circumference, and a second edge 4. The first edge 2 can be divided into a first portion 2a, which connects the joint surface 5 and the circumferential face 7, and a second portion 2b, which connects the joint surface 5 with the surface of the chamfered or rounded edge 9. The second edge 4 connects the surface of the chamfered or rounded edge 9 with the circumferential face 7.

Above noted maximum width is defined between the second edge portion 2b and the second edge 4. In the exemplary embodiment of the figures, the maximum width is substantially arranged in the middle between the ends of the chamfered or rounded edge 9. Nonetheless, it may also be situated at a different position but is preferably situated at a position, where impingement may most likely occur.

It should be noted that the first portion 2a of edge 2 may be slightly chamfered or rounded such as for reasons of production. However, such a first edge portion 2a generally has a constant width that is generally below 3 mm.

Fig. 2A shows a perspective view of a joint replacement member 1 according to an embodiment which is configured to be used with an intermediate member 500 for a joint replacement according to an embodiment shown in Fig. 2B.

The intermediate member 500 shown in Fig. 2B comprises a tissue attachment face 513 which is configured to be attached to native tissue such as bone tissue and/or surrounding soft tissue. The tissue attachment face 513 of the embodiment shown in Figs. 2A, 2B, 2C, 2D comprises a first protrusion 515 and a plurality of second protrusions 517 which are configured to enhance the fixation of the intermediate member 500 in the surrounding tissue. The combination of a first protrusion 515 and at least one second protrusion 517 particularly prevent the intermediate member from rotating about the first protrusion 515.

The intermediate member 500 further comprises a device attachment face 521 which is configured to mate with a corresponding part of the replacement member 1. The device attachment face 521 of the embodiment has a first through hole 525 which extends through the first protrusion 515 to the other side of the intermediate member 500, i. e. it penetrates the first protrusion 515 along the longitudinal direction of the first protrusion 515.

Further, second through holes 527 are extending from the device attachment face 521 into the second protrusions 517, such that the through holes 527 penetrate the device attachment face 521 and the second protrusions 517.

Fasteners, in particular screws, can be inserted through the first 525 and second 527 through holes, and, respectively, through the first 515 and second protrusions 517 in order to anchor the intermediate member 500 to bone tissue adjacent to the intermediate member 500.

The replacement member 1 has an attachment structure 11 that is configured to mate with a corresponding structure of the first hole 525. While it is noted that only the attachment structure 11 is illustrated in Figs. 2A and 2B, similar attachment structures may be provided for the interaction between the second through holes 527 of the intermediate member 500 with corresponding protrusions 13 on the replacement member 1.

In the embodiment shown in Figs. 2A and 2B, the attachment structure 11 is a snap-fit connection which is configured to snap into a corresponding connection part of the first through hole 525 (not shown). However, it is to be noted that other connections such as taper locks or other alternatives known to the person skilled in the art may be provided in order to secure the replacement member 1 to the intermediate member 500.

When implanted into a patient, the replacement member 1 and the intermediate member 500 are arranged such that the chamfered or rounded part of the edge is oriented caudally, that is, downwardly.

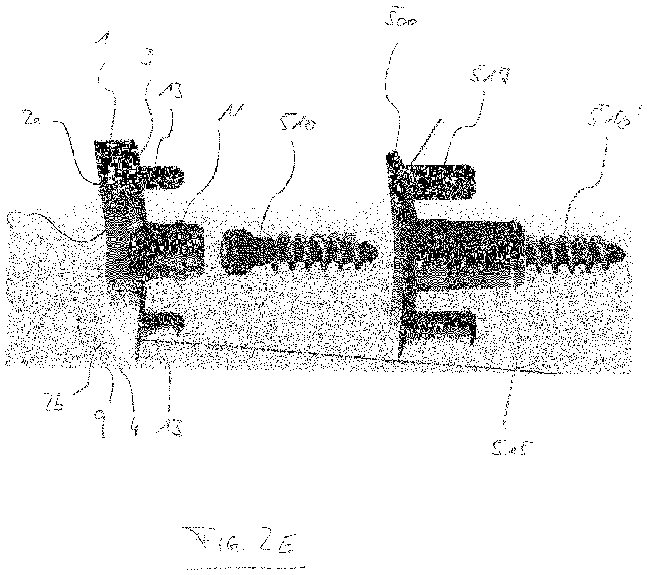

Fig. 2E shows an exploded view of an assembly of a replacement member 1 and an intermediate member 500 for implantation into a patient.

At first, the intermediate member 500 is attached to bone tissue, for example via a fixation member 510, 510', such as a screw, that is inserted into the first through hole (not visible) within the first protrusion 515. Then, the replacement member 1 is attached to the intermediate member 500, for example by means of a snap-fit connection between the attachment structure 11 and the first through hole (not visible).

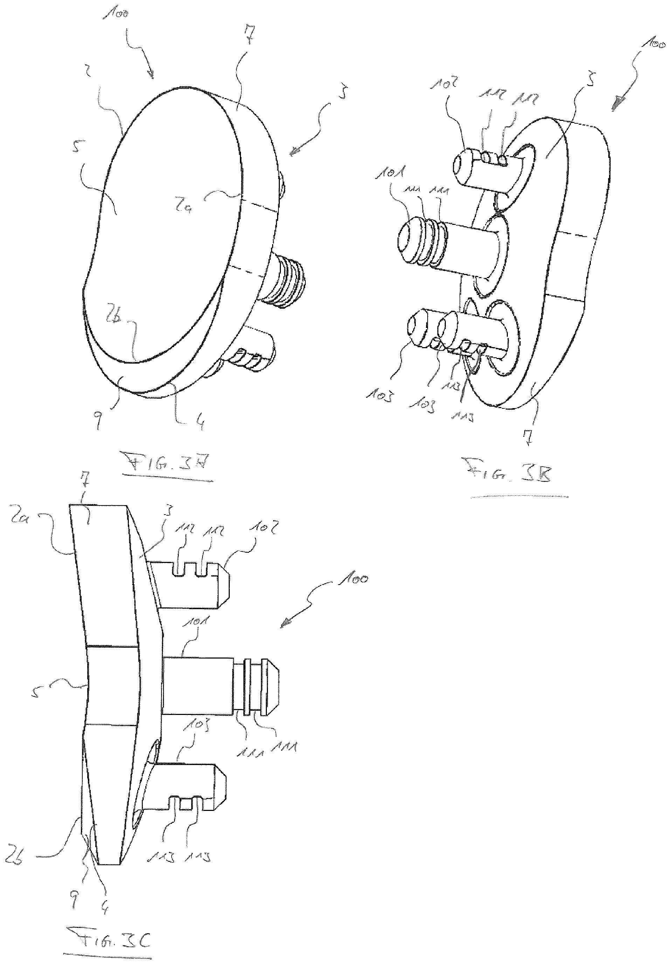

Figs. 3A, 3B and 3C show another embodiment of a replacement member 100 which is configured to be mounted directly into surrounding tissue without the interposition of an intermediate member.

Similar to the above, the replacement member 100 comprises a concave joint surface 5, a circumferential face 7, an attachment face 3 and a chamfered edge 9. As described above, the chamfered edge may also be a rounded edge. Like the edge of the previous embodiment, the chamfered or rounded edge 9is arranged between first and second edges 2, 4 of the joint surface 5 and the circumferential face 7, respectively.

While the configuration of the embodiment shown in Figs. 3A, 3B, and 3C is particularly similar to the configuration of the embodiment described with reference to Fig. 2A-E in respect to the side of the replacement member 1 comprising the joint surface 5, the replacement member 100 shown in Figs. 3A-C differs in the configuration of the attachment face 3.

The attachment face 3 of the embodiment shown in Fig. 3A-C is configured to be directly attached to surrounding tissue, either via bone cement or via bone ingrowth into the attachment face 3.

The attachment face 3 of the replacement member 100 is provided with a plurality of anchoring protrusions, in particular a first 101, a second 102, and a third 103 anchoring protrusion. In this exemplary embodiment, the substantially cylindrical first anchoring protrusion 101 is configured to extend substantially orthogonally from the attachment face 3.

As shown in Figs. 3, the first anchoring protrusion 101 may also be provided with annular recesses 111. It is to be noted that in this exemplary embodiment, the anchoring protrusion 101 comprises two annular recesses 111. However, any number of recesses 111 may be provided for anchoring of the replacement member 100. As shown in Figs. 3A-C, the recesses 111 may extend along the entire circumference of the anchoring protrusion 101. Naturally, they may also be configured to partially surround the anchoring protrusion 101.

Preferably, there is also at least a second anchoring protrusion 102 that extends from the attachment face 3 substantially parallel to the first anchoring protrusion 101. The plurality of anchoring protrusions generally extend parallel to each other into the same direction (direction of insertion).

As already discussed above, more than one anchoring protrusion prevents the replacement member 100 from rotating about a longitudinal axis of one of these protrusions. Alternatively, only one protrusion may be provided that is at least partly not formed with a circular cross-section so that once anchored in bone tissue, the geometry of the protrusion prevents the replacement member from rotating about the longitudinal axis of the protrusion. Naturally such a configuration of a protrusion may also be provided in case of multiple protrusions. This will be described in more detail below in relation to the partial recesses 112 and 113 of the second anchoring protrusion 102 and the third anchoring protrusion 103, respectively. It should be noted that the connection between the replacement member 1 and the intermediate member 500 may also be configured in this manner.

Further, the protrusions increase the surface provided for attaching the replacement member 100 to bone tissue. This is particularly advantageous for anchoring the replacement member by ingrowth of bone tissue.

The second anchoring protrusion 102 may be provided with, for example, two partial recesses 112. These partial recesses 112 are preferably configured to face outwardly in respect to the replacement member 100 as a whole. In other words, the recesses 112 partially extending along the circumference of the anchoring protrusion 102 are directed in a radial direction away from the first anchoring protrusion 101. Similar to the above, the recesses 112 are not particularly limited to the shape depicted in Fig. 3A-C, and may, for example, be configured annularly, similarly to the recess or recesses 111 of the first anchoring protrusion 101. Also, the number of the recesses 102 is not particularly limited to the number shown in the embodiment.

Besides increasing the surface for anchoring further, any number of these recesses formed in the protrusions result in a form fit that is particularly effective when using bone cement for anchoring the replacement member 100.

Further, there may also be at least one third anchoring protrusion 103 extending from the attachment face 3 and substantially parallel to the first anchoring protrusion 101. In Figs. 3A to 3C, two third anchoring protrusion 103 are provided with two partial recesses 113 each, which are also configured to face outwardly as previously described in relation to the second anchoring protrusion 102.

Similar to the above, the recess or recesses 103 is/are not particularly limited to the shape depicted in Figs. 3A-C, and may, for example, be configured annularly, similarly to the recess or recesses 111 of the first anchoring protrusion 101. Likewise, the number of recesses 103 is not particularly limited to the number shown in the exemplary embodiment of Figs. 3A-3C.

When the replacement member 100 is implanted, the second anchoring portion 102 is preferably arranged to be cranially, that is, on the side of an abduction movement of a shoulder joint. The third anchoring protrusions 103 are preferably located caudally, that is, on the side of an adduction movement and on the side opposite to the side, where the chamfered or rounded edge 9 is located.

Since the anatomy of the shoulder provides caudally more space than cranially, the embodiment shown in Figs. 3A-C comprises one second anchoring protrusion 102 cranially and two anchoring protrusions 103 caudally. The skilled person will appreciate that the present disclosure is not limited to the depicted configuration and the numbers of anchoring protrusions 101, 102, and 103 may be varied. Nevertheless, it is to be noted that the provision of two anchoring protrusions 103 allows to position said anchoring protrusions 103 offset from the straight line defined by the anchoring protrusions 101 and 102. This offset provides additional stability for the replacement member 100, for example, against an anteversion-/retroversion movement.

As mentioned above, the direction of extension of the anchoring protrusions 101, 102, 103 is essentially parallel so that the replacement member 100 can be smoothly inserted into a plurality of correspondingly shaped parallel cavities that have been prepared within the bone tissue of a patient.

The recesses 111, 112, 113 provide space for the ingrowth of tissue and, in case of cemented replacement members, for the cement. It is therefore apparent that, while the precise shape of the recesses 111, 112, 113 is not limited, it is still advantageous to provide the recesses 112, 113, that is, the recesses of the second 102 and third 103 anchoring protrusion, at least facing radially outwards in relation to the replacement member 100. This configuration improves the mechanical resistance of the replacement member against torque, which may, for example, result from the movement of the arm, such as an adduction or abduction.

The attachment face 3 of the embodiment shown in Figs. 3A-C is formed in a convex shape, which resembles the native shape of a joint cup, and, thus, reduces the amount of native tissue that needs to be removed before implantation of the replacement member. More specifically, only the cartilage tissue has to be removed completely, wherein the bone tissue may only be adapted to the shape of the attachment face 3.

### LIST OF REFERENCE SIGNS

- 1: replacement member
- 2: first edge
- 2a: first portion
- 2b: second portion
- 3: attachment face
- 4: second edge
- 5: joint surface
- 7: circumferential or peripheral face
- 9: chamfered or rounded edge
- 11: attachment structure
- 13: protrusion
- 100: replacement member
- 101: first anchoring protrusion
- 102: second anchoring protrusion
- 103: third anchoring protrusion
- 111: annular recess
- 112, 113: partial recess
- 500: intermediate member
- 510, 510': fastener
- 513: tissue attachment face
- 515: first protrusion
- 517: second protrusion
- 521: device attachment face
- 525: first through hole
- 527: second through hole

## Claims

1. A replacement member (1, 100) for a shoulder joint replacement comprising an attachment face (3) on one side of the member (1, 100), a concave joint surface (5) on a side of the replacement member (1, 100) opposite to the side of the attachment face (3), and a circumferential face (7) connecting the attachment face (3) and the joint surface (5),
wherein a portion of the concave joint surface (5) is connected to the circumferential face (7) via a chamfered or rounded edge (9).

2. The member (1, 100) according to claim 1,
wherein the chamfered or rounded edge (9) is configured to be located at an adductive portion of the joint replacement when the implant joint replacement is implanted in a body.

3. The member (1, 100) according to any of the preceding claims,
wherein the concave joint surface face (5) has a curved profile, the curved profile being spherical, ellipsoidal, or ovoid shaped.

4. The member (1, 100) according to any of the preceding claims,
wherein the attachment face (3) is configured so as to fixedly attach the member (1, 100) to bone tissue or to another replacement member.

5. The member (1, 100) according to any of the preceding claims,
wherein the member is a glenoid replacement or a humeral replacement.

6. The member (1, 100) according to any of the preceding claims,
wherein a section forming the joint surface (5) comprises a composition of Vitamin E and UHMWPE.

7. The member (1, 100) according to any of the preceding claims, wherein the concave joint surface (5) is configured to engage with a mating joint replacement member.

8. A shoulder joint replacement, comprising:
the member (1, 100) according to any of the preceding claims, and
a mating joint replacement member,
wherein the member (1, 100) and the mating joint replacement member engage via a portion of the convex joint surface (5) of the member (2, 100).

9. Shoulder joint replacement comprising a first replacement member, in particular a replacement member according to any one of the preceding claims, and a second replacement member, wherein:
the first replacement member comprises an attachment face on one side of the member, a concave joint surface on a side of the replacement member opposite to the side of the attachment face, and a circumferential face connecting the circumferential attachment face and the joint surface;
the second replacement member comprises a convex joint surface, the convex joint surface forming a ball joint with the concave joint surface of the first replacement member, and an outer surface distal and adjacent to the convex joint surface; and
the concave joint surface is partly connected to the circumferential face via a chamfered or rounded edge, the chamfered or rounded edge being arranged such that a clearance is provided between the first replacement member and the second replacement member when the two replacement members are arranged relatively to each other so as to form a predetermined adducted arrangement.
